# FASCICULE DE BREVET EUROPEEN

(11) **EP 0 977 550 B1**
(45) Date de publication et mention de la délivrance du brevet: **31.08.2005**
(21) Numéro de dépôt: 98914937.2
(22) Date de dépôt: 17.03.1998
(51) Int. Cl.: A61K 7/48, A61K 35/78, A61K 7/06

(54) **UTILISATION D'AU MOINS UN EXTRAIT D'IRVINGIA GABONENSIS DANS UN PRODUIT COSMETIQUE ET/OU PHARMACEUTIQUE**
VERWENDUNG VON MINDESTENS EINEM IRVINGIA GABONENSIS-EXTRAKT IN KOSMETISCHEN UND/ODER PHARMAZEUTISCHEN PRODUKTEN
USE OF AT LEAST ONE IRVINGIA GABONENSIS EXTRACT IN A COSMETIC AND/OR PHARMACEUTICAL PRODUCT

(30) Priorité: 14.04.1997 FR 9704732
(43) Date de publication de la demande: 09.02.2000
(73) Titulaire: Cognis France S.A., 31360 Saint Martory (FR)
(72) Inventeur: PAULY, Gilles, F-54000 Nancy (FR)
(74) Mandataire: Nuss, Pierre
(86) Numéro de dépôt international: PCT/FR1998/000538
(87) Numéro de publication internationale: WO 1998/046204

(56) Documents cités:
- US-A- 3 964 500
- E. N. ONYEIKE ET AL.: "Effect of heat-treatment and defatting on the proximate composition of some Nigerian local soup thickeners." FOOD CHEMISTRY, vol. 53, no. 2, 1995, GB, pages 173-175, XP002052760 cité dans la demande
- CHEMICAL ABSTRACTS, vol. 112, no. 26, 25 juin 1990 Columbus, Ohio, US; abstract no. 240425, XP002052300 & J. O. ONYECHI ET AL.: "the tabletting properties of dika fat lubricant" DRUG. DEV. IND. PHARM., vol. 16, no. 7, 1990, pages 1203-1216,

## Description

La présente invention concerne le domaine de la cosmétologie et de la pharmacologie, notamment les applications cutanées et capillaires, et a pour objet l'utilisation d'au moins un extrait d'Irvingia gabonensis dans un produit cosmétique et/ou pharmaceutique pour la peau et/ou les phanères, ainsi qu'un produit cosmétique et/ou pharmaceutique comprenant un tel ou de tels extraits(s).

Irvingia gabonensis (également appelé manguier sauvage) est un arbre répandu dans les forêts humides de l'Afrique Centrale et Occidentale, dont le fruit oblong possède un noyau dur entouré par une chair fibreuse et comestible et renfermant deux graines semblables à des amandes.

Ces graines, broyées et mélangées avec de l'eau, du poivre ou d'autres condiments, sont largement utilisées en Afrique comme épaississant pour la préparation de soupes.

La composition chimique de la graine d'Irvingia est, d'après la littérature, la suivante (selon OL OKE, Nutrition reports international, 1978, vol. 17 n° 3, 293-297 et ONYECHI E.N., Food chemistry, 1995, 53,2, 173-175) :
protéines : 8,8 - 10,6 %
lipides : 55 - 62,2 %
hydrates de carbone : 19,2 - 19,6 %
fibres : 8,2 %

L'huile de graines d'Irvingia gabonensis, qui est solide à température ambiante contient notamment 55 - 59 % d'acide myristique et 35 - 36,3 % d'acide laurique.

L'utilisation potentielle de la graisse d'Irvingia dans la préparation de margarine, d'huile de cuisine, de savons et de produits pharmaceutiques a été évoquée. Cette graisse a notamment été étudiée pour la préparation de suppositoires ou comme lubrifiant pour granulés ou comprimés.

De plus, l'utilisation des polysaccharides d'Irvingia gabonensis comme épaississant ou comme sources d'hydrates de carbone pour les personnes diabétiques dans des produits alimentaires, et comme épaississant ou liant dans l'industrie pharmaceutique, a été évoquée.

Or, les inventeurs de la présente invention ont constaté de manière inattendue et surprenante que, en dehors de leurs propriétés épaississantes connues, les extraits d'Irvingia gabonensis, notamment les fractions polysaccharidiques d'Irvingia gabonensis, pouvaient être directement utilisées dans des produits cosmétiques et pharmaceutiques à application externe et présentaient des propriétés immédiates plus variées et quantitativement plus importantes que les polysaccharides déjà utilisés actuellement dans les produits cosmétiques ou pharmaceutiques, à savoir des propriétés hydratantes, filmogènes, adoucissantes et restructurantes.

De même, il a été constaté que la fraction lipidique ou graisse pouvait également être utilisée dans de tels produits et présentait des propriétés particulières, telles qu'une bonne stabilité chimique et une faible coloration.

Ainsi, l'objet général de la présente invention consiste en l'utilisation d'au moins un extrait d'Irvingia gabonensis en tant qu'agent(s) actif(s) pour la préparation d'un produit cosmétique et/ou pharmaceutique à usage topique pour la peau et/ou les phanères, cet ou ces extrait(s) étant préférentiellement obtenu(s) à partir des graines de l'arbre Irvingia gabonensis.

Conformément à un premier mode de réalisation de l'invention, l'extrait utilisé est constitué par la fraction polysaccharidique des graines, le cas échéant sous forme d'extrait polysaccharidique brut.

Selon un deuxième mode de réalisation préféré de l'invention, l'extrait utilisé est constitué par une partie de la fraction polysaccharidique des graines, le cas échéant sous la forme d'un polysaccharide purifié.

Dans ce cas, la fraction polysaccharidique ou le polysaccharide est préférentiellement extrait(e) des graines de l'arbre Irvingia gabonensis par l'eau ou des solutions aqueuses à pH neutre, alcalin ou acide.

Enfin, conformément à un troisième mode de réalisation de l'invention, l'extrait utilisé peut être constitué par la fraction lipidique ou graisse de graines d'Irvingia gabonensis, seule ou associée avec la fraction ou une partie de la fraction polysaccharidique desdites graines.

Les procédés d'obtention et de préparation des différents extraits d'Irvingia gabonensis mentionnés ci-dessus font partie des connaissances générales de l'homme du métier dans le domaine de la cosmétologie et de la pharmacologie.

A titre d'exemples non limitatifs, on décrira ci-après différents procédés d'obtention possibles des extraits de graines d'Irvingia gabonensis, notamment graisse et polysaccharides, utilisés dans le cadre de la présente invention.

### Exempte 1 :

950 grammes de graines d'Irvingia gabonensis sont tout d'abord broyées puis extraites pendant une heure à température ambiante dans 3 litres d'hexane.

Après décantation pendant une nuit à température ambiante, la phase hexane est soutirée, filtrée et concentrée à l'évaporateur rotatif.

Après séchage en étuve, on obtient 415,6 grammes d'une huile solide de couleur jaune pâle.

Un litre de solvant est ensuite ajouté au réacteur et l'extraction est renouvelée pendant 2 heures à 45° C. La totalité de l'extrait est filtré et le filtrat traité comme précédemment : 182,7 grammes d'huile sont ainsi obtenus.

Le résidu est à nouveau extrait dans 2,50 litres d'hexane pendant 2 heures à 45° C et traité comme précédemment : 84,4 grammes d'huile sont finalement obtenus.

Le rendement total en huile brute de ce procédé est de 71,8 %.

L'huile brute d'Irvingia gabonensis peut être raffinée et désodorisée par les techniques habituelles connues de l'homme du métier pour donner une huile solide de couleur blanche et parfaitement inodore.

Le résidu insoluble à l'hexane est une poudre de couleur beige représentant 260 grammes soit 27 % en poids par rapport à la graine entière initiale.

### Exemple 2 :

100 grammes de résidus insolubles à l'hexane, obtenus avec le procédé décrit dans l'exemple 1, sont homogénéisés dans 1,5 litres d'eau distillée.

Une extraction est ensuite opérée pendant 3 heures à 70° C.

Après refroidissement, la suspension visqueuse est centrifugée pendant 15 minutes à 5000 g.

Le surnageant est filtré sur une toile nylon afin de retenir une faible couche lipidique.

790 ml de surnageant, beige, visqueux et comprenant 3,9 % d'extrait sec sont ainsi recueillis et constituent les polysaccharides bruts.

Ces polysaccharides sont précipités sous forme de fibres par addition sous agitation dans deux volumes d'éthanol absolu.

Ces fibres sont récupérées par filtration, essorées, lavées dans de l'éthanol, puis dans de l'acétone et finalement séchées à l'air, puis à l'étuve à 40° C.

15,1 grammes de polysaccharides sont ainsi obtenus soit un rendement de 4,1 % en poids par rapport à la graine entière initiale.

### Exemple 3 :

100 grammes de graines sont broyées, puis extraites dans deux litres d'eau, pendant deux heures à 60° C.

La solution est refroidie à 30° C, puis centrifugée pendant 15 minutes à 5000 g.

La phase supérieure (couche de graisse) est éliminée, la phase aqueuse est filtrée sur toile et 1,55 litres de surnageant sont recueillis.

Le surnageant est de nouveau centrifugé et les polysaccharides sont ensuite précipités dans deux volumes d'éthanol absolu, puis traités comme dans l'exemple 2.

4,2 grammes de polysaccharides sont ainsi obtenus soit un rendement de 4,2 % par rapport à la graine entière initiale.

Les extraits de graines d'Irvingia gabonensis obtenus notamment par l'un quelconque des procédés décrits ci-dessus, notamment les polysaccharides, trouveront leur utilisation dans les préparations de soin pour la peau et les phanères, en tant qu'agents émollients, adoucissants, réparateurs, hydratants et élastifiants.

Lesdits polysaccharides pourront être utilisés sous forme brute ou purifiée, sous forme anhydre ou de solutions aqueuses et incorporés dans les formes cosmétiques les plus diverses, telles que lotions, hydrogels, émulsions H/E et E/H, microémulsions, produits de soins cutanés, produits de soins capillaires, etc.

La présente invention a également pour objet un produit cosmétique et/ou pharmaceutique à usage externe pour la peau et/ou les phanères, caractérisé en ce qu'il comporte entre 0,05 % et 50,00 % en poids d'un extrait ou d'extraits de graines d'Irvingia gabonensis.

Selon un mode de réalisation particulier de l'invention, ledit produit cosmétique et/ou pharmaceutique peut avantageusement consister en une composition traitante comportant entre 0,05 % et 15,00 % en poids d'un extrait ou d'extraits de graines d'Irvingia gabonensis.

A titre d'exemples non limitatifs de réalisations pratiques de l'invention, on décrira ci-après différent(e)s produits ou préparations cosmétiques ou dermatologiques, comprenant un ou des extraits de graines d'Irvingia gabonensis.

### Exemple 1 :

Un produit cosmétique conforme à l'invention, sous forme d'émulsion de rinçage capillaire pourra, par exemple, présenter une composition pondérale, constituée à partir des fractions A et B suivantes, telle qu'indiquée ci-après.

### Fraction A :

- Stéarate de glycérol (et) Ceteareth 20 5,00 %
- Extrait lipidique de graines d'Irvingia gabonensis
   (préparé selon le procédé de l'exemple 1) 1,00 %

### Fraction B :

- Diméthicone Propyl PG Bétaine 3,00 %
- Chlorure de Behen trimonium 1,00 %
- Eau distillée 90,00 %
- Parfum qsp
- Conservateur qsp

Le procédé de préparation et de fabrication de l'émulsion précitée consiste essentiellement, après mélange de leurs constituants, à chauffer séparément les fractions A et B à environ 70° C, à réaliser le mélange de ces deux fractions et à l'homogénéiser et, enfin, à agiter le mélange résultant jusqu'à refroidissement à température ambiante.

### Exemple 2 :

Un produit cosmétique conforme à l'invention, sous forme de lait hydratant pour le corps (H/E) pourra, par exemple, présenter une composition pondérale, constituée à partir des fractions A et B suivantes, telle qu'indiquée ci-après.

### Fraction A :

- Phosphate de Trilaureth-4 1,00 %
- Stéarate de polyglycérol-2 PEG-4 4,00 %
- Huile de paraffine 3,00 %
- Isononanoate cétostéarylique 4,00 %
- Extrait lipidique de graines d'Irvingia gabonensis
   (préparé selon le procédé de l'exemple 1) 4,00 %
- Isostéarate d'isopropyle 4,00 %
- Antioxydant qsp

### Fraction B :

- Carbomer 0,30 %
- Acide citrique 0,25 %
- Glycérine 3,00 %
- NaOH(N) 1,50 %
- Eau distillée 74,50 %
- Conservateur qsp
- Extrait polysaccharidique de graines d'Irvingia gabonensis
   (préparé selon le procédé de l'exemple 2) 3,00 %
- Parfum qsp

Le procédé de préparation et de fabrication du lait hydratant précité consiste essentiellement à mélanger les constituants de la fraction grasse A et à les chauffer à 70° C, à y ajouter le Carbomer, à mélanger les autres constituants de la fraction aqueuse B et à les chauffer à 70° C, à verser la fraction B dans la fraction A et à agiter le mélange jusqu'à refroidissement à température ambiante et, enfin, à homogénéiser l'émulsion résultante.

### Exempte 3 :

Un produit cosmétique conforme à l'invention, sous forme de crème (H/E) hydratante, émolliente et adoucissante pour le visage pourra, par exemple, présenter une composition pondérale, constituée à partir des fractions A, B et C suivantes, telle qu'indiquée ci-après.

### Fraction A :

- Stéarate de glycérol (et) Steareth-25 (et) Ceteth-20 (et) alcool stéarylique 8,00 %
- Alcool stéarylique 1,00 %
- Triglycéride d'acide caprylique / caprique 3,00 %
- Diméthicone stéaroxylique 3,00 %
- Extrait lipidique de graines d'Irvingia gabonensis
   (préparé selon le procédé de l'exemple 1) 3,00 %

### Fraction B :

- Glycérol 3,00 %
- Extrait polysaccharidique de graines d'Irvingia gabonensis (préparé selon le procédé de l'exemple 2) 2,00 %
- Eau distillée 77,00 %

### Fraction C :

- Parfum qsp
- Conservateur qsp

Le procédé de préparation et de fabrication de la crème précitée consiste essentiellement à mélanger et à chauffer la fraction grasse A à 90° C, à préparer la fraction aqueuse B par agitation à 75° C, à verser la fraction A dans la fraction B sous agitation turbine, à y ajouter la fraction C, à stopper l'agitation turbine vers 50° C et à réaliser une agitation planétaire jusqu'à refroidissement à température ambiante.

Bien entendu, l'invention n'est pas limitée aux modes de réalisation décrits. Des modifications restent possibles, notamment du point de vue de la constitution des divers éléments ou par substitution d'équivalents techniques, sans sortir pour autant du domaine de protection de l'invention.

## Revendications

1. Utilisation d'au moins un extrait d'Irvingia gabonensis en tant qu'agent(s) actif(s) pour la préparation d'un produit pharmaceutique à usage topique pour la peau et/ou les phanères.

2. Utilisation non thérapeutique d'au moins un extrait d'Irvingia gabonensis comme ou dans un produit cosmétique.

3. Utilisation selon la revendication 1 ou 2, **caractérisée en ce que** ledit ou lesdits extrait(s) est (sont) obtenu(s) à partir des graines de l'arbre Irvingia gabonensis.

4. Utilisation selon la revendication 3, **caractérisée en ce que** l'extrait utilisé est constitué par la fraction polysaccharidique des graines, le cas échéant sous forme d'extrait polysaccharidique brut.

5. Utilisation selon la revendication 3, **caractérisée en ce que** l'extrait utilisé est constitué par une partie de la fraction polysaccharidique des graines, le cas échéant sous la forme d'un polysaccharide purifié.

6. Utilisation selon la revendication 5, **caractérisée en ce que** la fraction polysaccharidique ou le polysaccharide est extrait(e) des graines de l'arbre Irvingia gabonensis par l'eau ou des solutions aqueuses à pH neutre, alcalin ou acide.

7. Utilisation selon la revendication 1 ou 2, **caractérisée en ce que** l'extrait utilisé est constitué par la fraction lipidique ou graisse de graines d'Irvingia gabonensis, seule ou associée avec la fraction ou une partie de la fraction polysaccharidique desdites graines.

8. Produit cosmétique à usage externe pour la peau et/ou les phanères, **caractérisé en ce qu'**il comporte entre 0,05 % et 50,00 % en poids d'un extrait ou d'extraits de graines d'Irvingia gabonensis.

9. Produit cosmétique à usage externe pour la peau et/ou les phanères, **caractérisé en ce qu'**il consiste en une composition traitante comportant entre 0,05 % et 15,00 % en poids d'un extrait ou d'extraits de graines d'Irvingia gabonensis.

10. Produit pharmaceutique à usage externe pour la peau et/ou les phanères, **caractérisé en ce qu'**il comporte entre 0,05 % et 50,00 % en poids d'un extrait ou d'extraits de graines d'Irvingia gabonensis.

11. Produit pharmaceutique à usage externe pour la peau et/ou les phanères, **caractérisé en ce qu'**il consiste en une composition traitante comportant entre 0,05 % et 15,00 % en poids d'un extrait ou d'extraits de graines d'Irvingia gabonensis.

## Patentansprüche

1. Verwendung von mindestens einem Irvingia-gabonensis-Extrakt als Wirkstoff(e) für die Herstellung eines Arzneimittelprodukts zur topischen Anwendung für die Haut und/oder die Epithelanhanggebilde.

2. Nicht therapeutische Verwendung von mindestens einem Irvingia-gabonensis-Extrakt als oder in einem Kosmetikprodukt.

3. Verwendung nach Anspruch 1 oder 2, **dadurch gekennzeichnet, daß** der Extrakt bzw. die Extrakte aus Samen des Baums Irvingia gabonensis stammt bzw. stammen.

4. Verwendung nach Anspruch 3, **dadurch gekennzeichnet, daß** der verwendete Extrakt aus einer Polysaccharidfraktion der Samen besteht, gegebenenfalls in Form eines Polysaccharid-Rohextrakts.

5. Verwendung nach Anspruch 3, **dadurch gekennzeichnet, daß** der verwendete Extrakt aus einem Teil der Polysaccharidfraktion der Samen besteht, gegebenenfalls in Form eines gereinigten Polysaccharids.

6. Verwendung nach Anspruch 5, **dadurch gekennzeichnet, daß** die Polysaccharidfraktion oder das Polysaccharid aus Samen des Baums Irvingia gabonensis mit Wasser oder mit alkalischen, sauren oder pH neutralen wäßrigen Lösungen extrahiert wird.

7. Verwendung nach Anspruch 1 oder 2, **dadurch gekennzeichnet, daß** der verwendete Extrakt aus der Lipid- oder Fettfraktion von Irvingia-gabonensis-Samen allein oder gemeinsam mit der Polysaccharidfraktion oder einem Teil der Polysaccharidfraktion dieser Samen besteht.

8. Kosmetikprodukt zur äußerlichen Anwendung für die Haut und/oder die Epithelanhanggebilde, **dadurch gekennzeichnet, daß** es 0,05 Gew.-% bis 50,00 Gew.-% eines Irvingia-gabonensis-Samenextrakts bzw. von Irvingia-gabonensis-Samenextrakten aufweist.

9. Kosmetikprodukt zur äußerlichen Anwendung für die Haut und/oder die Epithelanhanggebilde, **dadurch gekennzeichnet, daß** es aus einer Wirkzusammensetzung mit 0,05 Gew.-% bis 15,00 Gew.-% eines Irvingia-gabonensis-Samenextrakts bzw. von Irvingia-gabonensis-Samenextrakten besteht.

10. Arzneimittelprodukt zur äußerlichen Anwendung für die Haut und/oder die Epithelanhanggebilde, **dadurch gekennzeichnet, daß** es 0,05 Gew.-% bis 50,00 Gew.-% eines Irvingia-gabonensis-Samenextrakts bzw. von Irvingia-gabonensis-Samenextrakten aufweist.

11. Arzneimittelprodukt zur äußerlichen Anwendung für die Haut und/oder die Epithelanhanggebilde, **dadurch gekennzeichnet, daß** es aus einer Wirkzusammensetzung mit 0,05 Gew.-% bis 15,00 Gew.-% eines Irvingia-gabonensis-Samenextrakts bzw. von Irvingia-gabonensis-Samenextrakten besteht.

## Claims

1. Use of at least one Irvingia gabonensis extract as an active ingredient or as active ingredients for the preparation of a pharmaceutical product for topical use on the skin and/or hair, teeth and nails.

2. Non-therapeutic use of at least one Irvingia gabonensis extract as or in a cosmetic product.

3. Use according to claim 1 or 2, **characterised in that** said extract(s) is/are obtained from Irvingia gabonensis tree seeds.

4. Use according to claim 3, **characterised in that** the extract used consists of the polysaccharide portion of the seeds, optionally in the form of raw polysaccharide extract.

5. Use according to claim 3, **characterised in that** the extract used consists of part of the polysaccharide portion of the grains, optionally in the form of a purified polysaccharide.

6. Use according to claim 5, **characterised in that** the polysaccharide portion or the polysaccharide is extracted from the Irvingia gabonensis tree grains by water or pH neutral, alkaline or acidic aqueous solutions.

7. Use according to claim 1 or 2, **characterised in that** the extract used consists of the lipid or fatty portion of the Irvingia gabonensis grains, alone or associated with the portion or part of the polysaccharide portion of said grains.

8. Cosmetic product for external use on the skin and/or hair, teeth and nails, **characterised in that** it comprises between 0.05 % and 50.00 % by weight of an extract or extracts of Irvingia gabonensis grains.

9. Cosmetic product for external use on the skin and/or hair, teeth and nails, **characterised in that** it consists of a treatment composition comprising between 0.05 % and 15.00 % by weight of an extract or extracts of Irvingia gabonensis grains.

10. Pharmaceutical product for external use on the skin and/or hair, teeth and nails, **characterised in that** it comprises between 0.05 % and 50.00 % by weight of an extract or extracts of Irvingia gabonensis grains.

11. Pharmaceutical product, for external use on the skin and/or hair, teeth and nails, **characterised in that** it consists of a treatment composition comprising between 0.05 % and 15.00 % by weight of an extract or extracts of Irvingia gabonensis grains.
